Europäisches Patentamt

European Patent Office

Office européen des brevets

(19)

(11) Publication number: **0 000 066**
**A1**

(12) **EUROPEAN PATENT APPLICATION**

(21) Application number: **78100121.9**

(22) Date of filing: **08.06.78**

(51) Int. Cl.²: **G 01 N 33/16, C 07 C 99/12**

(30) Priority: **10.06.77 US 805502**

(43) Date of publication of application:
**20.12.78 Bulletin 78/1**

(84) Designated Contracting States:
**CH DE GB LU NL SE**

(71) Applicant: **Warren-Teed Laboratories, Inc.,
582 W. Goodale Street,
Columbus, Ohio 43215 (US)**

(72) Inventor: **Imondi, Anthony Rocco,
110 Daniel Drive,
Westerville, Ohio 43081 (US)**

(72) Inventor: **McLaughlin, James Edward,
14 Terminal Avenue,
Erdenheim, Pennsylvania 19002 (US)**

(72) Inventor: **Udinsky, John Richard,
1126 McKean Road,
Ambler, Pennsylvania 19002 (US)**

(74) Representative: **Körner, Ekkehard, Dipl.-Ing.,
Patentanwälte Müller-Börner, Wey & Körner,
Widenmayerstrasse 49,
D-8000 München 22 (DE)**

(54) **Determination of aromatic aminocarboxylic acids.**

(57) The concentration of aromatic aminocarboxylic acids and their conjugates in body fluids is determined by treating the body fluid with a hydrolyzing agent, extracting said acid with an ion exchange resin, washing residual body fluids from the resin, eluting said acid from the resin with an acid solution of a complexing agent and determining the concentration of said acid by comparing the color of the extract with the color of solutions having known concentrations of said acid wherein the improvement comprises employing a non-acid hydrolyzing agent, for example, a base or an enzyme.

EP 0 000 066 A1

Croydon Printing Company Ltd.

## Determination of Aromatic Aminocarboxylic Acids

The present method for determining the concentration of aromatic amines is the well-known method of Bratton-Marshall (Bratton, A. C. and Marshall, E. K., Jr., J. Biol. Chem., 128:537, 1939). This procedure involves acid hydrolysis, diazotization of the free amino group, destruction of excess nitrous acid and coupling of the diazonium salt with N-(1-naphthyl)ethylene diamine to form an azo dye followed by determination of the absorbance of the sample and comparison with the absorbance of solutions having known concentrations of aromatic amines. The Bratton-Marshall procedure requires skilled technicians and costly laboratory equipment. The method also demands adherence to a rigid time schedule, using three freshly prepared color reagents, vortexing and the use of a spectrophotometer because of the reagents' limited color range and stability.

This invention relates to an improved method for determining the concentration of aromatic amines having a carboxylic acid group and a kit for conducting said method. Accordingly, this invention relates to a method for determining the concentration of aromatic aminocarboxylic acid in a body fluid which comprises treating a known quantity of body fluid with a known quantity of a hydrolyzing agent, extracting the aromatic aminocarboxylic acid with an ion exchange resin, washing the resin, then eluting said acid with an acidic solution of a color complexing agent and determining the content of the aromatic aminocarboxylic acid by comparison of its color with the color of known concentrations of the aromatic aminocarboxylic acid wherein the improvement comprises using a nonacidic hydrolysis agent. With this invention one can determine the concentration of an aromatic aminocarboxylic acid such as p-aminobenzoic acid, o-aminobenzoic acid, m-aminobenzoic acid, p-aminohippuric acid or the N-acetylated
conjugates of these acids such as o-, m- and p-acetamido-
benzoic acid and the like using workers of ordinary skill,
simple equipment and avoid many of the steps required by
the Bratton-Marshall method. The method for determining
concentrations of an aromatic aminocarboxylic acid comprises treating a known quantity of body fluid, such as
urine, saliva, blood, plasma and the like with a known
quantity of a non-acidic hydrolyzing agent, for example,
an enzyme such as N-deacetylase which occurs in avian
kidneys (R. H. Nimmo-Smith, Biochem. J. 75: 284 (1960)) or
a base including an alkali metal base including sodium
hydroxide, potassium hydroxide and the like; (the heat of
the reaction is sufficient to cause complete hydrolysis if
the reaction vessel is insulated) extracting said acid
with an anion exchange resin, for example, strongly basic,
weakly basic or intermediate basic anion exchange resins
such as cholestyramine; a copolymer of styrene-divinylbenzene with quaternary ammonium functionality; crosslinked polyvinylpyrrolidone; strongly basic polystyrenes
such as those sold under the tradename Amberlite® IRA-
410, BioRex®-9 and the like; also resins prepared from
epoxy polyamines; polyethyleneimine; weakly basic polystyrenes, or phenolic polyamines such as those sold under
the tradename Amberlite® IR-45 and the like, washing the
residue from the resin, eluting said acid with an acid
solution of a color complexing agent, for example, aromatic aldehydes, such as p-dimethylaminocinnamaldehyde,
vanillin and the like, hydroxy substituted aromatics such
as $\lambda$-naphthol and the like and, lastly, comparing the
color of the extract with the color of solutions having
known concentrations of said acid.

This method has been found to be extremely useful in
conjunction with a method for evaluating pancreatic enzyme
sufficiency described and claimed in U.S. Pat. No.
3,745,212.

It should be noted that any anion exchange resin can
be employed in this invention such as those disclosed in U.S. Pat. Nos. 2,354,671; 2,365,151; 2,402,384; 2,675,359 and 2,591,574 which patents are hereby incorporated by reference.

This invention includes a kit for carrying out the above described procedure. The kit comprises: (a) a disposable syringe for measuring body fluid; (b) an insulated vial containing a known quantity of base or enzyme; (c) optionally, a vial for dilution of basified materials; (d) a column containing an ion exchange resin; (e) a column holder; (f) a vial containing an acid solution of a color complexing agent and (g) means of determining concentration, for example, by comparison with colors of known concentrations such as by comparing with vials containing known amounts of the aromatic aminocarboxylic acid being determined or with a color chart depicting colors of known concentrations of the aromatic aminocarboxylic acid being determined or by determining optical densities of the above in a colorimeter.

Time-hydrolysis curves revealed that hydrolysis occurs most rapidly during the first 20 minutes after mixing the body fluid with the base. Since the decreased rate of catalysis parallels the loss of heat generated upon mixing, heat is a rate-limiting factor.

Insulation of the vial with Styrofoam proved to be an effective way of retaining the heat of the reaction. Use of a polystyrene vial made the insulation process more efficient. There is also reduced heat loss with decreased air space above the liquid. Complete hydrolysis is now possible within one hour at room temperature.

. An increase or decrease in the concentration of alkali adversely affected the rate and degree of hydrolysis. The ratio of body fluid to base or enzyme is in the range of from 1:3 to 3:1. When 50% aqueous sodium hydroxide was employed, a 1:1 ratio of urine to base was shown to be optimum. 1.5 ml. of body fluid contained enough acid for differentiation of concentration.

The time necessary for alkaline hydrolysis can be reduced with the introduction of heat. Studies showed that only 10 minutes at $100^{\circ}$C. is necessary for complete alkaline hydrolysis of 1.5 ml. of urine in 1.5 ml. of 50% aqueous base and can be an alternate method to room temperature hydrolysis. Therefore, the hydrolysis can be conducted at a temperature in the range of from about $35^\circ$ to about $100^\circ$C. for a period of time in the range of from about 10 minutes (at $100^\circ$C.) to about 60 minutes (at about $35^\circ$C.).

The concentration of the color complexing reagent is generally in the range of from about .001% to about 1.0% with a concentration of about 0.025% being preferred.

Para-aminobenzoic acid (PABA) is an excellent tracer molecule for many *in vivo* diagnostic tests. It is nontoxic, rapidly and well absorbed from the gastrointestinal tract and is excreted in the urine usually as a conjugated metabolite. This method to detect urinary PABA is applicable in other diagnostic tests using this same tracer.

Wolgemuth, et al., *Digestive Diseases*, Vol. 21, No. 9, p. 821 (1976), demonstrated a useful test for determining bacterial overgrowth in the upper gastrointestinal tract. PABA was conjugated with cholic acid to make an enzyme-labile bile acid conjugate. In the presence of enteric bacteria, PABA is split from the bile acid conjugate and is readily absorbed and excreted in the urine. The amount of PABA recovered is an effective and reliable index of the extent of bacterial overgrowth in animal models. Urinary excretion of PABA and its metabolites is also useful in the diagnosis of liver disease (Whittlesey, *Johns Hopkins Hospital Bulletin* 100:51 (1957)).

Para-aminohippuric acid (PAH) is frequently used in renal function studies. PAH-clearance tests are used to measure renal blood flow and to diagnose congestive heart failure, hypertension, renal arterio- and glomerulosclerosis, polycystic disease, pyelonephritis and renal hyperplasia or malignancy.

<div align="center">Reagents</div>

1. Sodium hydroxide - 50% aqueous solution;
2. Acidified solution of p-dimethylaminocinnamaldehyde;
3. Rosin column and

4. Aromatic aminocarboxylic acid standards.

The following illustrates the procedure using urine as the body fluid in the determination of PABA:

## Alkaline Hydrolysis

1. Using the syringe, aspirate exactly 1.5 ml. of urine and deliver into a plastic vial containing 1.5 ml. of 50% aqueous sodium hydroxide solution.

2. Cap the vial and shake ten times to hydrolyze (reaction generates heat).

3. Immediately insulate the vial by inserting into a Styrofoam manifold and wrapping the entire system with a sheet of glass wool. Allow to stand undisturbed for at least one hour.

## Extraction

4. After the hydrolysis, quantitatively transfer the hydrolysate to a second, larger vial. Rinse the first vial three times with water and add this to the second vial. (This is an optional step as the hydrolysis step can be conducted in a larger vial.)

5. Fill the remainder of the second vial with water to bring to about 20 ml. Cap and mix the vial.

6. Snap off the top and bottom tabs of a disposable ion-exchange column containing the resin and support the column by a plastic holder.

7. Carefully pour the solution of the second vial into the column reservoir and allow the liquid to slowly percolate through the resin. This liquid is discarded.

8. Rinse the resin by filling the column reservoir twice with water. Allow each volume to drain completely.

9. Remove the column from the holder and shake off any excess water.

## Elution

10. Pour an acidified solution of 0.025% p-dimethylaminocinnamaldehyde into the column reservoir and collect in a third vial.

11. Cap and shake the third vial. The resulting solution will be colored a shade of orange or rose-red.

## Quantitation

12. Compare the series of colored standard solutions or a chart depicting same with the unknown sample. View all samples from directly above the opening, looking toward the bottom of each vial. (It is important that this is done under good lighting.)

13. Match the unknown with the standard that most closely approximates its color. If the unknown is greater than the 50% standard (more red), it is normal. Values equal to, or less than, 50% should be confirmed by a second, outside test.

The method for determining aromatic aminocarboxylic acids described above is fast, accurate and reproducible. The method simplifies the presently employed Bratton-Marshall procedure and can be carried out employing low cost, disposable equipment.

1. In a method for determining the concentration of aromatic aminocarboxylic acid in a body fluid which comprises treating a known quantity of body fluid with a known quantity of a hydrolyzing agent, extracting the aromatic aminocarboxylic acid with an ion exchange resin, washing the resin, then eluting said acid with an acidic solution of a color complexing agent and determining the content of the aromatic aminocarboxylic acid by comparison of its color with the color of known concentrations of the aromatic aminocarboxylic acid wherein the improvement comprises using a hydrolysis agent comprising an alkali metal base.

2. The method of claim 1 wherein the nonacidic hydrolysis is conducted at a temperature in the range of from $35^{\circ}$ to $100^{\circ}$C. for a period of time in the range of from 60 to 10 minutes.

3. The method of claim 1 wherein the base is a 50% aqueous sodium hydroxide solution.

4. The method of claim 1 wherein the aromatic aminocarboxylic acid is selected from p-aminobenzoic acid, o-aminobenzoic acid, m-aminobenzoic acid or p-aminohippuric acid or their N-acetylated conjugates.

5. The method of claim 1 wherein the body fluid is urine.

6. The method of claim 5 for determining p-aminobenzoic acid (PABA) or conjugated metabolites thereof in urine which comprises treating a measured sample of urine with a measured quantity of 50% aqueous sodium hydroxide, maintaining the temperature at about $35^{\circ}$ to $40^{\circ}$C. for

one hour, diluting the basic solution with water; then
adsorbing the PABA on an ion exchange resin, washing the
resin, eluting the PABA with an acidified 0.025% solution
of p-dimethylaminocinnamaldehyde and finally determining
the concentration of PABA or conjugated metabolites.

7.  A kit for carrying out the method of claim 1
which comprises:
      a.  a syringe;
      b.  a vial containing a known quantity of alkali
metal base;
      c.  optionally, a vial for dilution;
      d.  an ion exchange resin column;
      e.  a column holder;
      f.  a vial containing an acidic solution of a
          color complexing agent and
      g.  a means for determining the concentration
          of an aromatic aminocarboxylic acid.

8.  The kit of claim 7 which comprises:
      a.  a disposable syringe of 1-3 ml. capacity;
      b.  a disposable vial containing 1.5 ml. of
          50% aqueous sodium hydroxide;
      c.  a disposable column containing an ion
          exchange resin;
      d.  a disposable column holder;
      e.  a disposable vial containing an acidic solution of 0.025% of p-dimethylaminocinnamaldehyde and
      f.  means for determining concentration selected
          from vials containing known concentrations or
          a chart depicting the colors of known concen-
          trations of the aromatic aminocarboxylic acid
          being determined.

## DOCUMENTS CONSIDERED TO BE RELEVANT

| Category | Citation of document with indication, where appropriate, of relevant passages | Relevant to claim |
|---|---|---|
| A | CHEMICAL ABSTRACTS, vol. 67, nr. 11, 11 september 1967, Columbus Ohio, USA C.D.SCOTT a.o. "Automatic, high resolution analysis of urine for its ultra violet-absorbing constituents", page 4.770, second column, abstract 50948u  \* Abstract \* | 1 |
| A | CHEMICAL ABSTRACTS, vol. 82, nr. 1, 6 january 1975, Columbus Ohio, USA W.H.WAUGH a.o. "Simplified measurement of p- aminohippurate and other arylamines in plasma and urine", page 163, second column, abstract nr. 1.759j  \* Abstract \* | 1 |

**CLASSIFICATION OF THE APPLICATION (Int. Cl.²)**

G 01 N 33/16
C 07 C 99/12

**TECHNICAL FIELDS SEARCHED (Int.Cl.²)**

G 01 N 33/16

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 18-09-1978 | MEYLAERTS |